# EUROPEAN PATENT SPECIFICATION

(11) **EP 4 678 146 B1**
(45) Date of publication and mention of the grant of the patent: **09.09.2026**
(21) Application number: 25185568.0
(22) Date of filing: 26.06.2025
(51) Int. Cl.: A61F 5/01

(54) **HALLUX VALGUS CORRECTION BRACE**
HALLUX-VALGUS-KORREKTURSPANGE
ATTELLE DE CORRECTION D'HALLUX VALGUS

(30) Priority: 12.07.2024 JP 2024112538
(43) Date of publication of application: 14.01.2026
(73) Proprietor: Actment Co., Ltd., Kasukabe-shi Saitama 344-0057 (JP)
(72) Inventor: Ogawa, Akira, Kasukabe-shi, Saitama, 344-0057 (JP); Shimizu, Shingo, Koshigaya-shi, Saitama, 343-8540 (JP)
(74) Representative: Grünecker Patent- und Rechtsanwälte PartG mbB

(56) References cited:
- CN-U- 219 332 136
- JP-A- 2006 051 168
- JP-U- 3 105 647
- KR-B1- 102 646 350

## Description

### TECHNICAL FIELD

The present invention relates to a hallux valgus correction brace.

### BACKGROUND ART

A foot disease called hallux valgus, in which the big toe bends toward the second toe, has been known and there exists a hallux valgus correction brace for a conservative treatment of the hallux valgus. The hallux valgus correction brace is a brace that supports the toes with a gap being maintained between the big toe and the second toe, and various forms of the hallux valgus correction brace are known (see, for instance, Patent Literature 1: Japanese Utility Model Registration No. 3105647, and Patent Literature 2: Japanese Utility Model Registration No. 3068807).

The above-described hallux valgus correction brace is intended for daily and long-term wearing for the purpose of alleviating the pain associated with hallux valgus. However, a typical hallux valgus correction brace, which is usually made of silicone rubber as in Patent Literatures 1 and 2, is likely to deteriorate over time due to friction, dirt, or the like. Thus, the typical hallux valgus correction brace has a difficulty in withstanding daily and long-term wearing, necessitating costs and efforts for replacement.

A hallux valgus correction brace in accordance with the preamble of independent claim 1 is disclosed in CN219332136U.

### SUMMARY OF THE INVENTION

An object of the invention is to provide a hallux valgus correction brace that maintains a gap between a big toe and a second toe and withstands daily and long-term wearing.
[1] A hallux valgus correction brace according to an aspect of the invention includes: a first insertion piece configured to receive a big toe of a foot; a second insertion piece configured to receive a second toe or a middle toe of the foot; and a gap maintainer coupling the first insertion piece and the second insertion piece, the gap maintainer being configured to apply an elastic force to the first insertion piece and the second insertion piece in a direction away from each other, in which the first insertion piece, the second insertion piece, and the gap maintainer are integrally formed by an elongated member made of metal.
[2] In the above aspect of [1], it is preferable that a material of the elongated member include a nickel-titanium alloy.
[3] In the above aspect of [2], it is preferable that a shape recovery temperature of the nickel-titanium alloy be 32 degrees C or less.
[4] In the above aspect of any one of [1] to [3], it is preferable that assuming that a direction in which the first insertion piece and the second insertion piece are arranged side by side is defined as a foot-width direction and an insertion direction of the second toe or the middle toe into the second insertion piece is defined as a foot-length direction, the first insertion piece and the second insertion piece each form a groove opening on one side in an orthogonal direction orthogonal to each of the foot-width direction and the foot-length direction.
[5] In the above aspect of [4], it is preferable that the first insertion piece and the second insertion piece each include an inner end and an outer end forming a pair of edges of the groove, and the gap maintainer couple the inner end of the first insertion piece and the inner end of the second insertion piece.
[6] In the above aspect of [5], it is preferable that the gap maintainer be disposed on the one side in the orthogonal direction with respect to the outer end of at least one of the first insertion piece or the second insertion piece.
[7] In the above aspect of [5] or [6], it is preferable that the gap maintainer include: a first projection connected to the inner end of the first insertion piece and projecting toward the groove of the first insertion piece; a second projection connected to the inner end of the second insertion piece and projecting toward the groove of the second insertion piece; and a coupler coupling the first projection and the second projection.
[8] In the above aspect of [7], it is preferable that the first projection and the second projection each have an arc shape.
[9] In the above aspect of [7] or [8], it is preferable that the coupler have an arc shape curved to project on an other side in the orthogonal direction.
[10] In the above aspect of any one of [4] to [6], it is preferable that the gap maintainer have an arc shape curved to project on the one side in the orthogonal direction.
[11] The above aspect of any one of [4] to [8] may further include one or more protective members covering at least an inner surface of the groove of each of the first insertion piece and the second insertion piece.
[12] In the above aspect of any one of [4] to [11], it is preferable that the elongated member have a plate shape with a predetermined width and a predetermined thickness as being unfolded in the foot-width direction.
[13] In the above aspect of [12], the elongated member may have a mesh shape with a plurality of through holes.

### BRIEF DESCRIPTION OF DRAWINGS

Fig. 1 is a perspective view illustrating a hallux valgus correction brace of a first exemplary embodiment.
Fig. 2 is a side view illustrating the hallux valgus correction brace of the first exemplary embodiment.
Fig. 3 illustrates a wearing example of the hallux valgus correction brace of the first exemplary embodiment.
Fig. 4 illustrates the wearing example in Fig. 3 viewed from the opposite side.
Fig. 5 illustrates another wearing example of the hallux valgus correction brace of the first exemplary embodiment.
Fig. 6 illustrates the wearing example in Fig. 5 viewed from the opposite side.
Fig. 7 exemplifies deformation of the hallux valgus correction brace of the first exemplary embodiment in use.
Fig. 8 is a graph exemplifying a nickel-titanium alloy.
Fig. 9 is a perspective view illustrating a hallux valgus correction brace of a second exemplary embodiment.
Fig. 10 is a side view illustrating the hallux valgus correction brace of the second exemplary embodiment.
Fig. 11 exemplifies deformation of the hallux valgus correction brace of the second exemplary embodiment in use.
Fig. 12 is a perspective view illustrating a hallux valgus correction brace according to a modification.
Fig. 13 is a perspective view illustrating a hallux valgus correction brace according to another modification.
Fig. 14 is a perspective view illustrating a hallux valgus correction brace according to still another modification.

### DETAILED DESCRIPTION

A plurality of exemplary embodiments of the invention will be described on the basis of the drawings.

### First Exemplary Embodiment

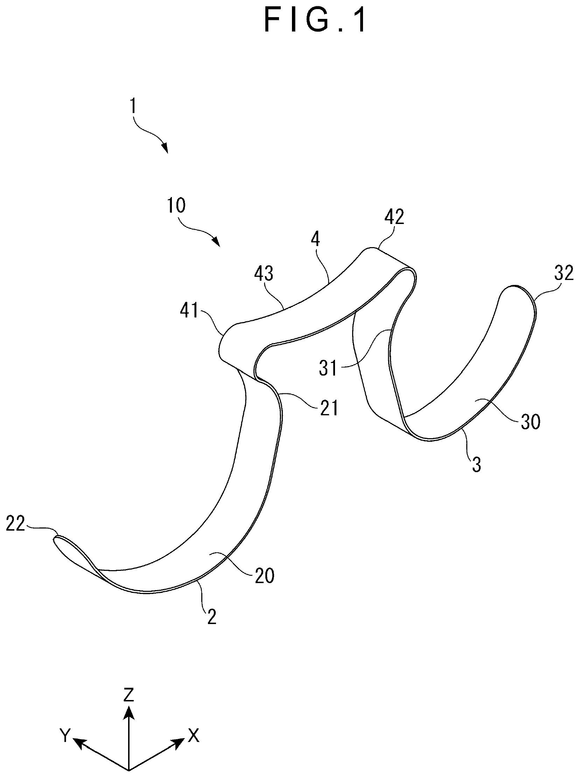

A hallux valgus correction brace 1 according to a first exemplary embodiment of the invention will be described with reference to Fig. 1 to Fig. 7. The hallux valgus correction brace 1 of the exemplary embodiment is used by being worn on a foot F of a user for a conservative treatment of the hallux valgus of the user.

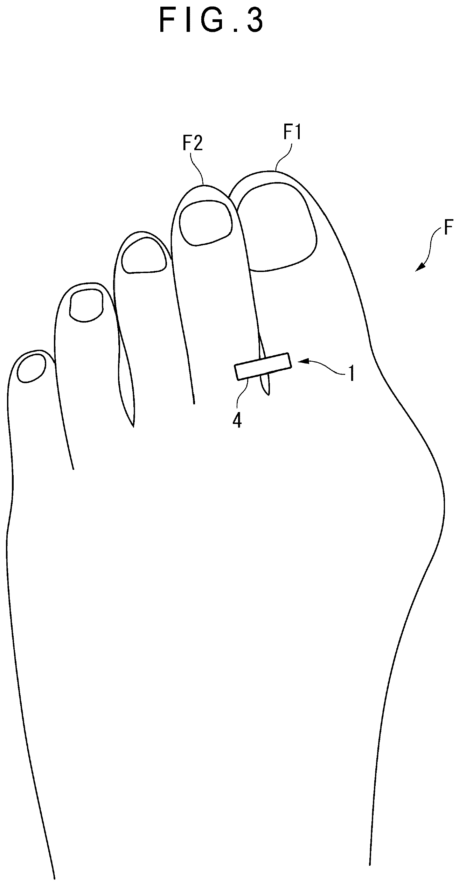

The hallux valgus correction brace 1 includes a first insertion piece 2 that receives a big toe F1 of the foot F, a second insertion piece 3 that receives a second toe F2 of the foot F, and a gap maintainer 4 coupling the first insertion piece 2 and the second insertion piece 3 and configured to apply an elastic force to the first insertion piece 2 and the second insertion piece 3 in a direction away from each other (see, for instance, Fig. 1 and Fig. 3).

An X-direction, a Y-direction, and a Z-direction that are mutually orthogonal are used in the following description. During the use of the hallux valgus correction brace 1, the X-direction corresponds to a foot-width direction, the Y-direction corresponds to a foot-length direction (i.e., an insertion direction of the second toe F2 in the second insertion piece 3). The Z-direction corresponds to an orthogonal direction orthogonal to each of the foot-width direction and the foot-length direction. It should be noted that a +Z-direction and a -Z-direction referred to in the exemplary embodiment each correspond to either an instep direction or a sole direction in accordance with a wearing direction of the hallux valgus correction brace 1. Since the first insertion piece 2, the gap maintainer 4, and the second insertion piece 3 are arranged side by side in the X-direction in this order, a direction toward a center of the gap maintainer 4 in the X-direction is referred to as a direction inward in the X-direction and an opposite direction thereto is referred to as a direction outward in the X-direction.

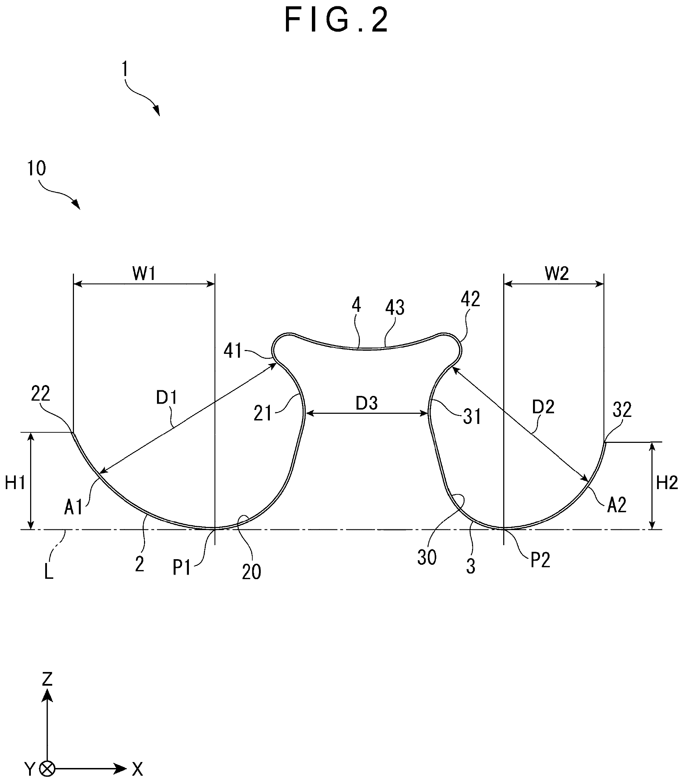

The hallux valgus correction brace 1 of the exemplary embodiment (i.e., the first insertion piece 2, the second insertion piece 3, and the gap maintainer 4) is integrally formed by an elongated member 10. The elongated member 10, which has a shape illustrated in Fig. 1 and Fig. 2, has a plate shape with a length in the X-direction, a width in the Y-direction, and a thickness in the Z-direction as being unfolded in the X-direction. In other words, the elongated member 10 of the exemplary embodiment is produced as a member in the shape illustrated in Fig. 1 and Fig. 2 by applying a molding process or the like to a plate-shaped material.

A length in the X-direction of the elongated member 10 when the elongated member 10 is unfolded in the X-direction is preferably in a range from 80 to 130 mm and is, for instance, 100 mm.

A width (a Y-directional dimension) of the elongated member 10 is preferably in a range from 3 to 10 mm and is, for instance, in a range from 4 to 6 mm.

A thickness (a Z-directional dimension) of the elongated member 10 is preferably in a range from 0.1 to 0.6 mm and is, for instance, 0.2 mm.

### Configuration of Hallux Valgus Correction Brace 1

A specific configuration of the hallux valgus correction brace 1 of the exemplary embodiment will be described with referenced to Fig. 1 and Fig. 2.

The first insertion piece 2 forms a groove 20 opening on one side in the Z-direction (a +Z-side in the exemplary embodiment). Moreover, the first insertion piece 2 includes an inner end 21 and an outer end 22, which provide a pair of edges on both sides of the groove 20 in the X-direction, respectively. The first insertion piece 2 is curved to form the groove 20 while extending in the X-direction from the inner end 21 to the outer end 22.

The second insertion piece 3 is disposed away from the first insertion piece 2. The second insertion piece 3 forms a groove 30 opening on one side in the Z-direction (the +Z-side in the exemplary embodiment). Moreover, the second insertion piece 3 includes an inner end 31 and an outer end 32, which provide a pair of edges on both sides of the groove 30 in the X-direction, respectively. The second insertion piece 3 is curved to form the groove 30 while extending in the X-direction from the inner end 31 to the outer end 32.

Here, the first insertion piece 2 has a curved shape corresponding to the big toe F1 of the foot F and the second insertion piece 3 has a curved shape corresponding to the second toe F2 of the foot F. For instance, assuming that a tangent L in the X-direction is in contact with each of the curved shapes of the first insertion piece 2 and the second insertion piece 3 as illustrated in Fig. 2, a curvature radius R1 of the first insertion piece 2 on the tangent L is larger than a curvature radius R2 of the second insertion piece 3 on the tangent L.

Further, an X-directional dimension W1 from a point P1 (a contact point with the tangent L), which is a portion closest to a -Z-side of the first insertion piece 2, to the outer end 22 of the first insertion piece 2 is larger than an X-directional dimension W2 from a point P2 (a contact point with the tangent L), which is a portion closest to the -Z-side of the second insertion piece 3, to the outer end 32 of the second insertion piece 3. Furthermore, a Z-directional dimension H1 from the outer end 22 of the first insertion piece 2 to the tangent L is larger than a Z-directional dimension H2 from the outer end 32 of the second insertion piece 3 to the tangent L.

It should be noted that the curved shape of each of the first insertion piece 2 and the second insertion piece 3 is not limited to an arc and may have a distortion corresponding to a peripheral shape of each toe.

The gap maintainer 4 couples the inner end 21 of the first insertion piece 2 and the inner end 31 of the second insertion piece 3. The gap maintainer 4 is disposed on the +Z-side with respect to the outer end 22 of the first insertion piece 2 and the outer end 32 of the second insertion piece 3.

Specifically, the gap maintainer 4 includes a first projection 41 connected to the inner end 21 of the first insertion piece 2, a second projection 42 connected to the inner end 31 of the second insertion piece 3, and a coupler 43 coupling the first projection 41 and the second projection 42.

The first projection 41 forms an arc projecting toward the groove 20 of the first insertion piece 2 (outward in the X-direction) while extending in the +Z-direction from the inner end 21 of the first insertion piece 2. The first projection 41 is thus opposed to a part of an inner surface of the groove 20. Here, an opposed portion A1, which is opposed to the first projection 41, of the groove 20 is preferably located between the outer end 22 of the first insertion piece 2 and the above-described point P1. A connection portion between the first projection 41 and the inner end 21 of the first insertion piece 2 has a gently curved shape.

The second projection 42 forms an arc projecting toward the groove 30 of the second insertion piece 3 (outward in the X-direction) while extending in the +Z-direction from the inner end 31 of the second insertion piece 3. The second projection 42 is thus opposed to a part of an inner surface of the groove 30. Here, an opposed portion A2, which is opposed to the second projection 42, of the groove 30 is preferably located between the outer end 32 of the second insertion piece 3 and the above-described point P2. A connection portion between the second projection 42 and the inner end 31 of the second insertion piece 3 has a gently curved shape.

The arc shapes of the first projection 41 and the second projection 42 of the exemplary embodiment have respective curvature radii R3 equal to each other, but the curvature radii R3 may be different. The respective curvature radii R3 of the first projection 41 and the second projection 42 are smaller than the curvature radius R1 of the first insertion piece 2 and the curvature radius R2 of the second insertion piece 3. Moreover, a dimension D1 connecting the first projection 41 and the opposed portion A1 of the groove 20 is larger than a dimension D2 connecting the second projection 42 and the opposed portion A2 of the groove 30.

The coupler 43 extends along the X-direction and couples the first projection 41 and the second projection 42. The coupler 43 of the exemplary embodiment has an arc shape curved to slightly project in the -Z-direction but may be linear. A curvature radius R4 of the coupler 43 is larger than the respective curvature radii R3 of the first projection 41 and the second projection 42.

Moreover, a dimension in the X-direction of the coupler 43 is set to maintain a gap D3 in the X-direction between the first insertion piece 2 and the second insertion piece 3. The gap D3, which is set in accordance with a desired correction force for making a gap between the big toe F1 and the second toe F2, is preferably in a range from 7 to 20 mm and is, for instance, in a range from 12 to 15 mm. The correction force increases with an increase in the gap D3, whereas the correction force decreases with a decrease in the gap D3. A distance between the above-described points P1 and P2 is determined depending on the gap D3 and is, for instance, in a range from 10 to 20 mm.

### Method of Using Hallux Valgus Correction Device 1

An exemplary method of using the hallux valgus correction brace 1 of the exemplary embodiment will be described with reference to Fig. 3 to Fig. 6. It should be noted that although the foot F of the user exemplified in Fig. 3 to Fig. 6 is a left foot, the same hallux valgus correction brace 1 can be worn on either the left or right foot by inverting the hallux valgus correction brace 1 so that positive and negative signs are interchanged in each of the X-direction and the Y-direction.

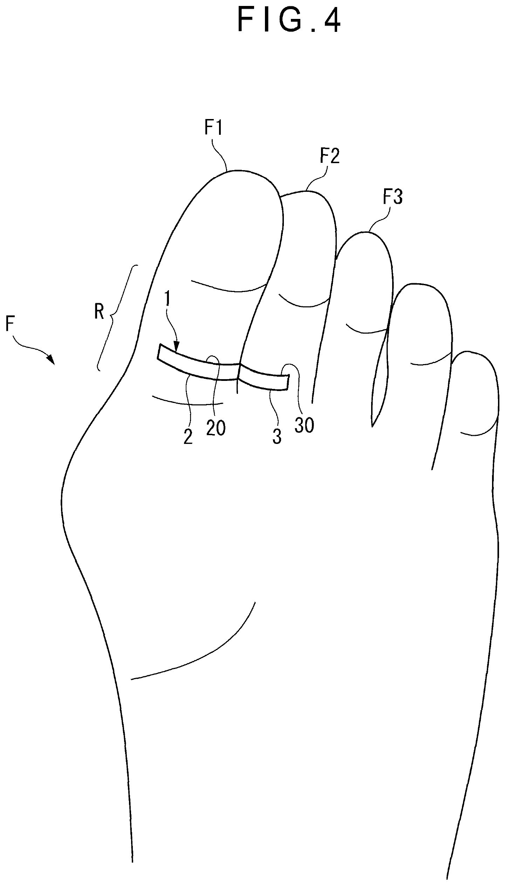

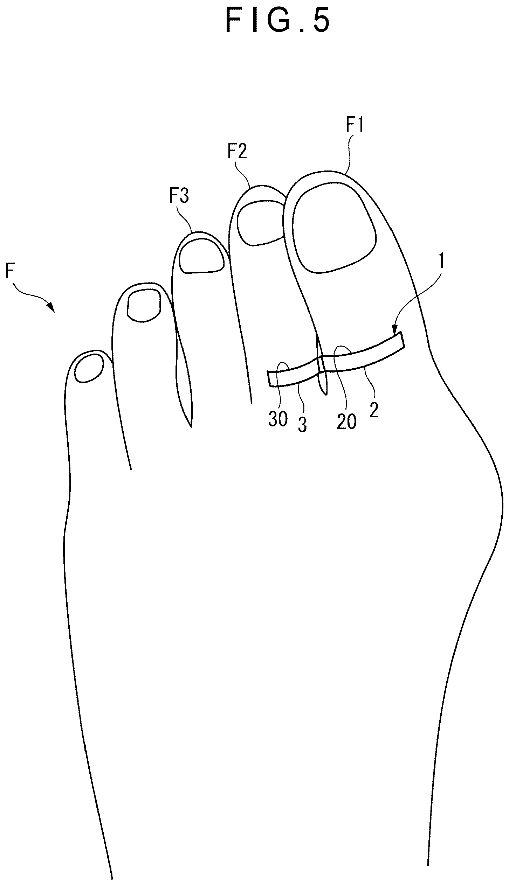

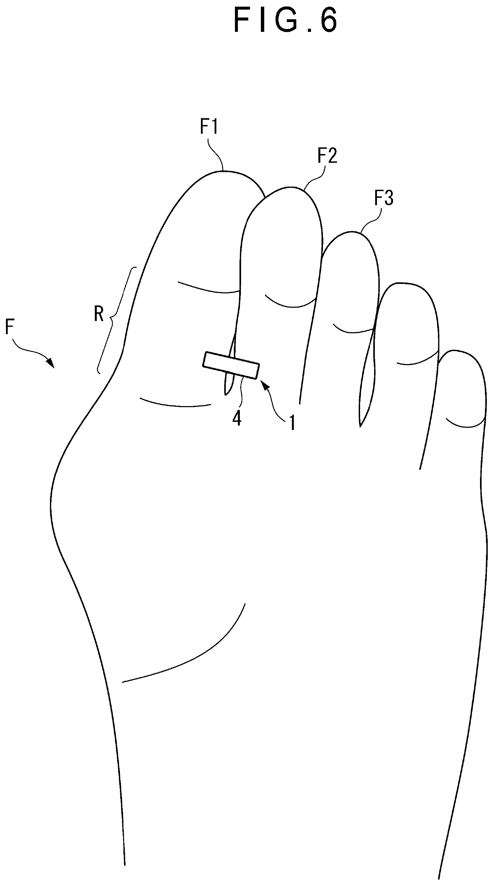

When the hallux valgus correction brace 1 is worn on the foot F of the user, the gap maintainer 4 is placed on an instep side of the toes of the foot F, while the first insertion piece 2 and the second insertion piece 3 are placed on a back side of the toes of the foot F as illustrated in Fig. 3 and Fig. 4. Alternatively, the gap maintainer 4 may be placed on the back side of the toes of the foot F, while the first insertion piece 2 and the second insertion piece 3 may be placed on the instep side of the toes of the foot F as illustrated in Fig. 5 and Fig. 6.

In either case, the big toe F1 is inserted in the groove 20 of the first insertion piece 2 and sandwiched between the first projection 41 and the opposed portion A1 to be held. Likewise, the second toe F2 is inserted in the groove 30 of the second insertion piece 3 and sandwiched between the second projection 42 and the opposed portion A2 to be held. The gap maintainer 4 is placed between the big toe F1 and the second toe F2. At this time, the first insertion piece 2 and the second insertion piece 3 are fitted on a section R corresponding to a proximal phalanx of each toe of the foot F and are prevented from positional displacement with the assistance of neighboring joints (see Fig. 4 and Fig. 6).

It should be noted that, as a condition of the hallux valgus of the user, Fig. 3 and Fig. 4 exemplify a condition in which the big toe F1 is bent to overlap a lower side of the second toe F2, and Fig. 5 and Fig. 6 exemplify a condition in which the big toe F1 is bent to overlap an upper side of the second toe F2. A combination of the condition of the hallux valgus and a wearing orientation of the hallux valgus correction brace 1 is not particularly limited and a different combination is possible.

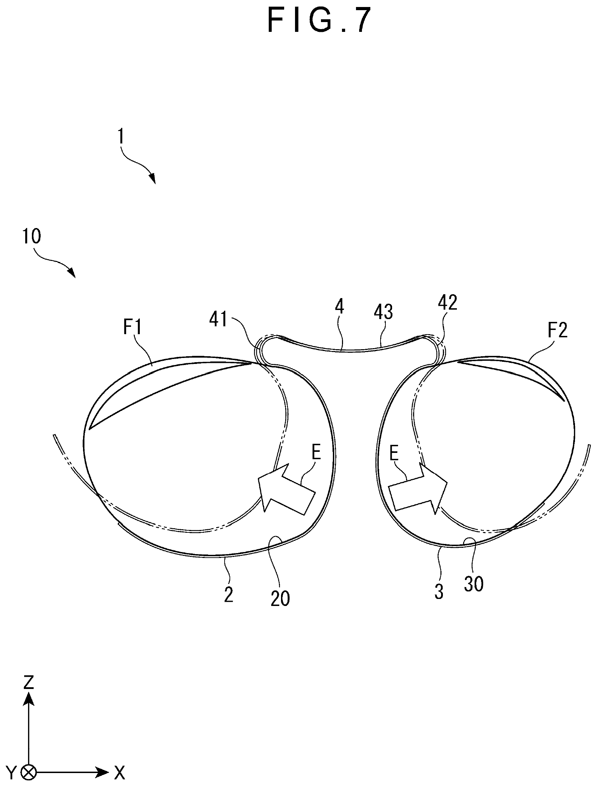

In Fig. 7, dash-double-dot lines indicate the hallux valgus correction brace 1 before being worn on the foot F and solid lines indicate the hallux valgus correction brace 1 being worn on the foot F. As illustrated in Fig. 7, the hallux valgus correction brace 1 receives a load from each of the big toe F1 and the second toe F2 when being worn on the foot F of the user. At this time, the gap maintainer 4 is elastically deformed so that the first insertion piece 2 and the second insertion piece 3 approach each other. In particular, in the exemplary embodiment, the first insertion piece 2 pivots around the first projection 41 and the second insertion piece 3 pivots around the second projection 42, causing the first insertion piece 2 and the second insertion piece 3 to approach each other.

The gap maintainer 4 thus applies, as an elastic force in a direction for the first insertion piece 2 and the second insertion piece 3 to move away from each other, an elastic force E containing not only an X-directional component but also a Z-directional component. The elastic force of the X-directional component of the gap maintainer 4 is allowed to function as a correction force to maintain a gap between the big toe F1 and the second toe F2. The elastic force of the Z-directional component of the gap maintainer 4 is allowed to function as a correction force for eliminating a displacement in the Z-direction of the big toe F1 overlapping the second toe F2.

It should be noted that in the exemplary embodiment, the big toe F1 and the second toe F2 are held by different portions of the hallux valgus correction brace 1 as described above, which makes it possible to prevent the big toe F1 from getting under the second toe F2 or getting on the second toe F2.

### Material of Elongated Member 10

A material of the elongated member 10 constituting the hallux valgus correction brace 1 of the exemplary embodiment may be a metal such as stainless steel, and is preferably nickel-titanium alloy. Nickel-titanium alloy, which is also referred to as Nitinol, is an alloy containing Ni and Ti at atomic contents approximately equal to each other and may also contain any additive element such as Co, Cu, or Nb.

In the exemplary embodiment, a shape recovery temperature of a nickel-titanium alloy constituting the elongated member 10 is preferably 32 degrees C or less. It should be noted that the shape recovery and/or the shape recovery temperature of the nickel-titanium alloy can be determined by adjustment of a blending ratio of Ni and Ti and adjustment of a memory treatment temperature.

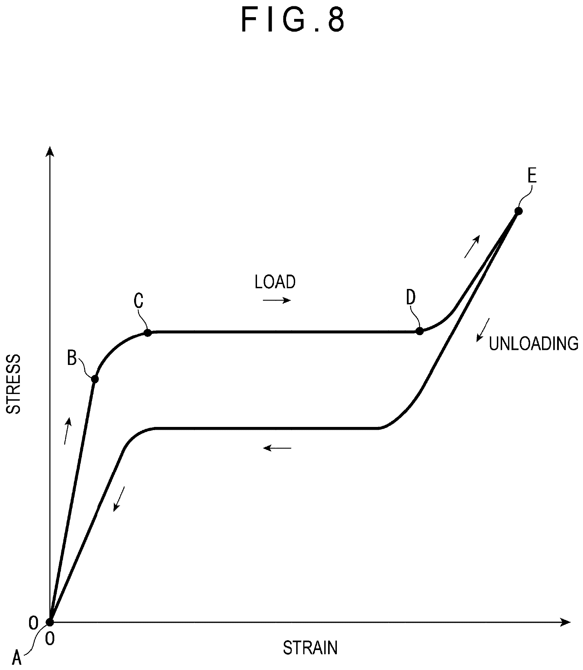

Properties of the nickel-titanium alloy at a higher temperature than the shape recovery temperature will be described below with reference to Fig. 8. Fig. 8 is a graph exemplifying a stress-strain curve of a superelastic nickel-titanium alloy.

First, the superelastic nickel-titanium alloy has a crystal structure called austenitic phase. In Fig. 8, the nickel-titanium alloy in the austenitic phase is elastically deformed in a linear manner with the strain being proportional to the stress between a point A and a point B. Then, while the strain increases with transformation from the austenite phase to a stress-induced martensitic phase between a point C and a point D, the stress is substantially constant. A region from the point C to the point D is referred to as a plateau region.

The nickel-titanium alloy exhibits a property called "superelasticity", which enables instant recovery to an original shape even after a relatively large deformation. It should be noted that an elastic strain of a typical metal is approximately 1%, whereas an elastic limit of nickel-titanium alloy is as wide as 6% to 10%, including the plateau region.

The hallux valgus correction brace 1 of the exemplary embodiment uses the above-described superelasticity of the nickel-titanium alloy, enabling the big toe F1 and the second toe F2 to move away from each other by virtue of, in particular, the elasticity in the plateau region.

### Effects of First Exemplary Embodiment

As described above, the hallux valgus correction brace 1 of the exemplary embodiment includes the first insertion piece 2 that receives the big toe F1 of the foot F, the second insertion piece 3 that receives the second toe F2 of the foot F, and the gap maintainer 4 coupling the first insertion piece 2 and the second insertion piece 3 and configured to apply an elastic force to the first insertion piece 2 and the second insertion piece 3 in a direction away from each other. The first insertion piece 2, the second insertion piece 3, and the gap maintainer 4 are integrally formed by the elongated member 10 made of metal.

In the above-described configuration, the first insertion piece 2, the second insertion piece 3, and the gap maintainer 4 are integrally formed by the elongated member 10 made of metal such as nickel-titanium alloy or stainless steel, so that it is easy to provide an elastic force functioning as a correction force and aging deterioration due to friction, dirt, or the like with use is less likely to occur. This configuration enables the hallux valgus correction brace 1 of the exemplary embodiment to maintain a gap between the big toe F1 and the second toe F2 of the foot and withstand daily and long-term wearing. As a result, costs and efforts for replacement are reduced.

In the exemplary embodiment, the material of the elongated member 10 is preferably nickel-titanium alloy and the shape recovery temperature of the nickel-titanium alloy is preferably 32 degrees C or less.

Such a configuration allows the properties of the nickel-titanium alloy, in particular, the superelastic property of the nickel-titanium alloy, to be used as the correction force of the hallux valgus correction brace 1.

For instance, the wide elastic limit of the nickel-titanium alloy enables the elongated member 10 to be elastically deformed over a wide range in accordance with a degree of deformity of the big toe F1 of the user, which is applicable also to a large deformity of the big toe F1 in a severe case. Moreover, the presence of the plateau region of the nickel-titanium alloy keeps substantially a constant stress to be applied to the big toe F1 and the second toe F2 of the user, thus preventing an excessive force from being applied thereto, even though a large strain is generated in the elongated member 10. Therefore, it is possible to help correct the severely deformed big toe F1 while reducing a pain of the user caused by the correction.

In the exemplary embodiment, the first insertion piece 2 and the second insertion piece 3 are respectively provided with the grooves 20 and 30 opening on one side in the orthogonal direction (in the +Z-direction) orthogonal to each of the foot-width direction and the foot-length direction.

Such a configuration facilitates the formation of the first insertion piece 2 and the second insertion piece 3 by the elongated member 10. The first insertion piece 2 and the second insertion piece 3 may be worn on either the instep side or the back side of the toes of the foot F. That is, the user is allowed to select the wearing orientation of the hallux valgus correction brace 1 in accordance with individual wearing comfort.

In the exemplary embodiment, the first insertion piece 2 and the second insertion piece 3 include the inner ends 21 and 31 and the outer ends 22 and 32, which form the respective pairs of edges of the grooves 20 and 30, and the gap maintainer 4 couples the inner end 21 of the first insertion piece 2 and the inner end 31 of the second insertion piece 3.

In such a configuration, the first insertion piece 2 and the second insertion piece 3 approach each other while pivoting around the gap maintainer 4 during the elastic deformation of the gap maintainer 4. The gap maintainer 4 thus applies not only the elastic force of the X-directional component but also the elastic force of the Z-directional component to the first insertion piece 2 and the second insertion piece 3. In other words, the hallux valgus correction brace 1 of the exemplary embodiment is allowed to apply the correction force not only in the X-direction but also in the Z-direction to the toes of the foot F. It is thus possible to favorably correct a deformity where the big toe F1 overlaps the upper or lower side of the second toe F2. As a result, a contact condition of the user's sole against the ground can be improved and also the user's knee pain can be alleviated.

In the exemplary embodiment, the gap maintainer 4 is disposed on one side (the +Z-side) in the orthogonal direction orthogonal to each of the foot-width direction and the foot-length direction, with respect to the outer ends 22 and 32 of the first insertion piece 2 and the second insertion piece 3.

When the user wears the hallux valgus correction brace 1, such a configuration can reduce a feeling of discomfort given to the user by inhibiting the outer ends 22 and 32 of the first insertion piece 2 and the second insertion piece 3 from excessively projecting from the foot F.

In the exemplary embodiment, the gap maintainer 4 includes the first projection 41 connected to the inner end 21 of the first insertion piece 2 and projecting toward the groove 20 of the first insertion piece 2, the second projection 42 connected to the inner end 31 of the second insertion piece 3 and projecting toward the groove 30 of the second insertion piece 3, and the coupler 43 coupling the first projection 41 and the second projection 42.

In such a configuration, the big toe F1 is allowed to be held as sandwiched between the first projection 41 and the opposed portion A1 of the first insertion piece 2. Likewise, the second toe F2 is allowed to be held as sandwiched between the second projection 42 and the opposed portion A2 of the second insertion piece 3. This configuration makes it possible to reliably hold the big toe F1 and the second toe F2, thus favorably preventing the big toe F1 from getting under the second toe F2 or getting on the second toe F2.

In the exemplary embodiment, the first projection 41 and the second projection 42 each have an arc shape.

Such a configuration allows the elastic force of each of the first projection 41 and the second projection 42 of the gap maintainer 4 to be favorably used as the correction force to be applied to the toes of the foot F.

In the exemplary embodiment, the coupler 43 has a curved shape curved to project on the other side (the -Z-side) in the orthogonal direction orthogonal to each of the foot-width direction and tch a configuration allohe foot-length direction.

Suws not only the elastic force of each of the first projection 41 and the second projection 42 of the gap maintainer 4 but also the elastic force of the coupler 43 to be favorably used as the correction force to be applied to the toes of the foot F.

In the exemplary embodiment, the elongated member 10 has a plate shape with a predetermined width and a predetermined thickness as being unfolded in the foot-width direction (the X-direction).

Such a configuration allows the hallux valgus correction brace 1 to be favorably produced using a plate-shaped material of the elongated member 10.

### Second Exemplary Embodiment

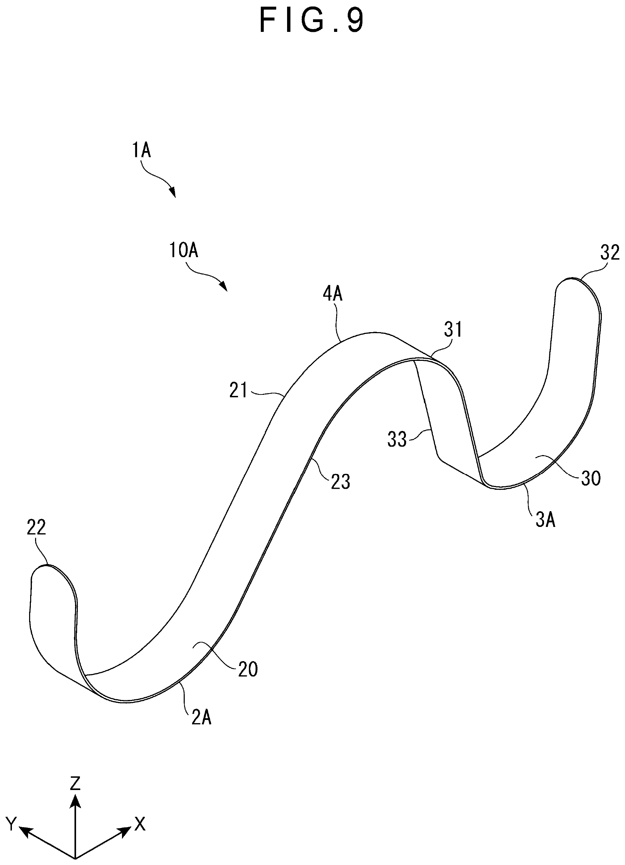

A hallux valgus correction brace 1A according to a second exemplary embodiment of the invention will be described with reference to Fig. 9 to Fig. 11. It should be noted that the hallux valgus correction brace 1A of the exemplary embodiment is suitable for use for a conservative treatment of a severer hallux valgus (a condition in which the deformity of a big toe is larger) than in the first exemplary embodiment.

Specifically, the hallux valgus correction brace 1A of the second exemplary embodiment includes a first insertion piece 2A that receives the big toe F1 of the foot F, a second insertion piece 3A that receives a middle toe F3 of the foot F, and a gap maintainer 4A coupling the first insertion piece 2A and the second insertion piece 3A and configured to apply an elastic force to the first insertion piece 2A and the second insertion piece 3A in a direction away from each other.

Hereinbelow, the hallux valgus correction device 1A of the second exemplary embodiment will be described, focusing on the differences from the hallux valgus correction device 1 of the first exemplary embodiment. For components similar to those of the first exemplary embodiment, the like reference codes will be used as in the first exemplary embodiment and the explanation thereof will be omitted or simplified.

The hallux valgus correction brace 1A (i.e., the first insertion piece 2A, the second insertion piece 3A, and the gap maintainer 4A) of the second exemplary embodiment is integrally formed by an elongated member 10A, as with the hallux valgus correction brace 1 of the first exemplary embodiment.

A dimension of the elongated member 10A is substantially similar to that of the first exemplary embodiment. Specifically, a length in the X-direction of the elongated member 10A as unfolded in the X-direction is preferably in a range from 80 to 130 mm and is, for instance, 105 mm. A width (a Y-directional dimension) of the elongated member 10A is preferably in a range from 3 to 10 mm and is, for instance, 8 mm. A thickness (a Z-directional dimension) of the elongated member 10A is preferably in a range from 0.1 to 0.6 mm and is, for instance, 0.3 mm.

A material of the elongated member 10A may be a metal such as stainless steel and is preferably nickel-titanium alloy as in the first exemplary embodiment.

The first insertion piece 2A and the second insertion piece 3A have substantially similar shapes to those of the first insertion piece 2 and the second insertion piece 3 of the first exemplary embodiment, respectively. That is, the first insertion piece 2A and the second insertion piece 3A form the grooves 20 and 30 opening on one side (the +Z-side) in the Z-direction, respectively. The first insertion piece 2A and the second insertion piece 3A include the inner end 21 and the outer end 22 and the inner end 31 and the outer end 32, respectively, which provide respective pairs of edges on both sides in the X-direction of the grooves 20 and 30.

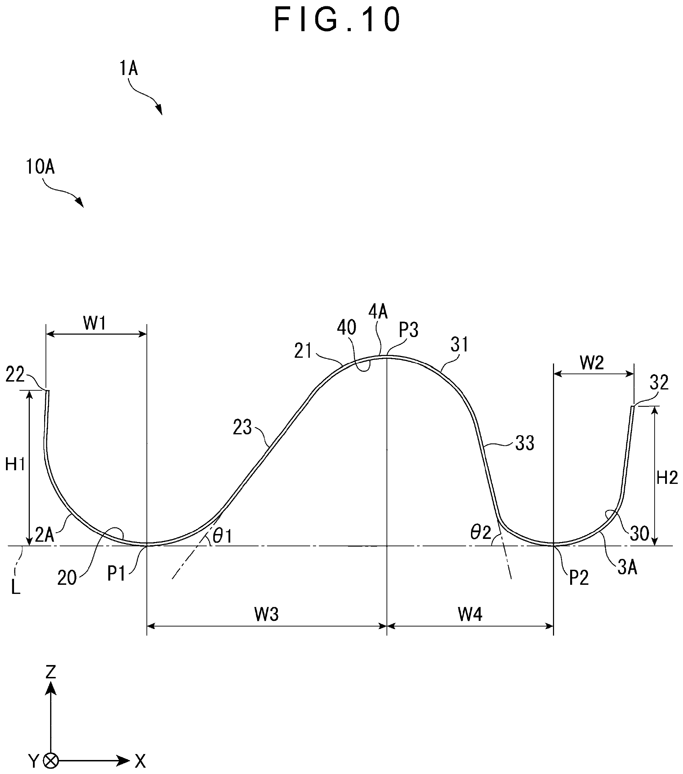

The first insertion piece 2A includes an extended portion 23, which extends outward in the X-direction and toward the -Z-side from the inner end 21 of the first insertion piece 2A and is inclined with respect to each of the X-direction and the Z-direction as illustrated in Fig. 10. Likewise, the second insertion piece 3A includes an extended portion 33, which extends outward in the X-direction and toward the -Z-side from the inner end 31 of the second insertion piece 3A and is inclined with respect to each of the X-direction and the Z-direction.

Here, similarly to the first exemplary embodiment, the tangent L in the X-direction that is in contact with each of curved shapes of the first insertion piece 2A and the second insertion piece 3A is assumed. It is preferable that an inclination angle θ1 of the extended portion 23 relative to the tangent L be smaller than an inclination angle θ2 of the extended portion 33 relative to the tangent L. It should be noted that the inclination angles θ1 and θ2 are not limited to the angles exemplified in Fig. 10 and may be set in a range from 0 degrees to 90 degrees as desired.

Similarly to the first exemplary embodiment, the point P1 (a contact point with the tangent L), which is a portion closest to the -Z-side of the first insertion piece 2A, and the point P2 (a contact point with the tangent L), which is a portion closest to the -Z-side of the second insertion piece 3A, are assumed. The X-directional dimension W1 from the point P1 of the first insertion piece 2A to the outer end 22 of the first insertion piece 2A is larger than the X-directional dimension W2 from the point P2 of the second insertion piece 3A to the outer end 32 of the second insertion piece 3A.

Moreover, the Z-directional dimension H1 from the outer end 22 of the first insertion piece 2A to the tangent L is larger than the Z-directional dimension H2 from the outer end 32 of the second insertion piece 3A to the tangent L.

The gap maintainer 4A has a different shape from the gap maintainer 4 of the first exemplary embodiment. Specifically, the gap maintainer 4A has an arc shape curved to project in the +Z-direction while being smoothly continuous with the inner ends 21 and 31 of the first insertion piece 2A and the second insertion piece 3A. In other words, the gap maintainer 4A forms a groove 40 opening in the -Z-direction, together with the extended portions 23 and 33 of the first insertion piece 2A and the second insertion piece 3A.

Here, a point P3, which is a portion closest to the +Z-side of the gap maintainer 4A, is assumed. It is preferable that a curvature radius R5 on the point P3 of the gap maintainer 4A be smaller than the curvature radius R1 of the first insertion piece 2A on the tangent L and larger than the curvature radius R2 of the second insertion piece 3A on the tangent L. Moreover, it is preferable that an X-directional dimension W3 from the point P1 of the first insertion piece 2A to the point P3 of the gap maintainer 4A be larger than an X-directional dimension W4 from the point P2 of the second insertion piece 3A to the point P3 of the gap maintainer 4A. The total of the X-directional dimensions W3 and W4, i.e., a distance between the points P1 and P2 is, for instance, in a range from 40 to 55 mm.

It should be noted that the curvature radius R5 of the gap maintainer 4A and the X-directional dimensions W3 and W4 are not limited to those exemplified in Fig. 10, and may be set as desired along with the above-described inclination angles θ1 and θ2.

### Method of Using Hallux Valgus Correction Device 1A

Similarly to the hallux valgus correction brace 1 of the first exemplary embodiment, by inverting the hallux valgus correction brace 1A of the second exemplary embodiment so that the positive and negative signs are interchanged in each of the X-direction and the Y-direction, the same hallux valgus correction brace 1A can be worn on either the left or right foot. Moreover, when the hallux valgus correction brace 1A is worn on the foot F of the user, the gap maintainer 4A may be placed on the instep side of the foot F or placed on the back side of the foot F.

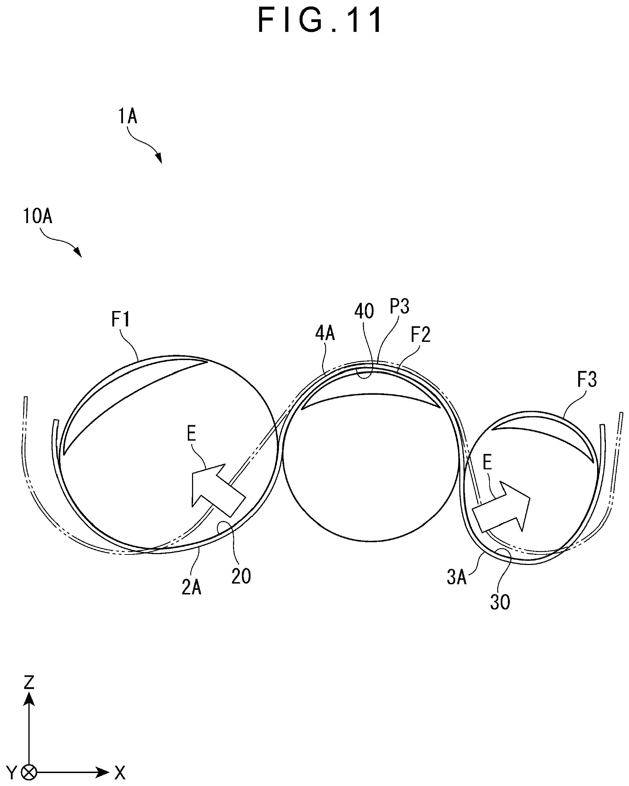

Fig. 11 exemplifies the hallux valgus correction brace 1A worn on the foot F. In Fig. 11, dash-double-dot lines indicate the hallux valgus correction brace 1A before being worn on the foot F and solid lines indicate the hallux valgus correction brace 1A being worn on the foot F.

As illustrated in Fig. 11, when the hallux valgus correction brace 1A is worn on the foot F of the user, the big toe F1 is inserted in the groove 20 of the first insertion piece 2A, the second toe F2 is inserted in the groove 40 of the gap maintainer 4A, and the middle toe F3 is inserted in the groove 30 of the second insertion piece 3A. At this time, the hallux valgus correction brace 1A receives a load from each of the big toe F1, which is deformed toward the second toe F2, and the middle toe F3. The gap maintainer 4A is elastically deformed accordingly so that the first insertion piece 2A and the second insertion piece 3A approach each other. In particular, in the exemplary embodiment, the first insertion piece 2A and the second insertion piece 3A each pivot around the point P3 of the gap maintainer 4A, causing the first insertion piece 2A and the second insertion piece 3A to approach each other.

The gap maintainer 4A thus applies, as an elastic force in a direction for the first insertion piece 2A and the second insertion piece 3A to move away from each other, the elastic force E containing not only an X-directional component but also a Z-directional component. The X-directional component of the elastic force of the gap maintainer 4A is allowed to function as a correction force to maintain a gap between the big toe F1 and the second toe F2 and a gap between the second toe F2 and the middle toe F3. The Z-directional component of the elastic force of the gap maintainer 4A is allowed to function as a correction force for eliminating a displacement in the Z-direction of the big toe F1 overlapping the second toe F2.

It should be noted that the hallux valgus correction brace 1A of the exemplary embodiment may be used in a similar manner to the first exemplary embodiment. Specifically, the big toe F1 is inserted in the groove 20 of the first insertion piece 2A and the second toe F2 is inserted in the groove 30 of the second insertion piece 3A with the warped gap maintainer 4A being placed between the big toe F1 and the second toe F2. In this usage as well, the elastic force of the gap maintainer 4A serves as a correction force for the big toe F1, and a gap can be maintained between the big toe F1 and the second toe F2.

### Effects of Second Exemplary Embodiment

The hallux valgus correction brace 1A of the exemplary embodiment is able to produce an effect similar to that of the above-described first exemplary embodiment. Moreover, the hallux valgus correction brace 1A of the exemplary embodiment uses not only the second toe F2 but also the middle toe F3 to support the big toe F1, thus enabling reliable support even in a case of a large deformity of the big toe F1. Therefore, it is possible to more favorably prevent the big toe F1 from getting under the second toe F2 or getting on the second toe F2.

### Modifications

The invention is not limited to the above-described exemplary embodiments and may encompass modifications described below within a scope in which the object of the invention is achievable.
(1) The hallux valgus correction braces 1 and 1A of the above-described exemplary embodiments may further include one or more protective members 5 provided on the elongated members 10 and 10A.

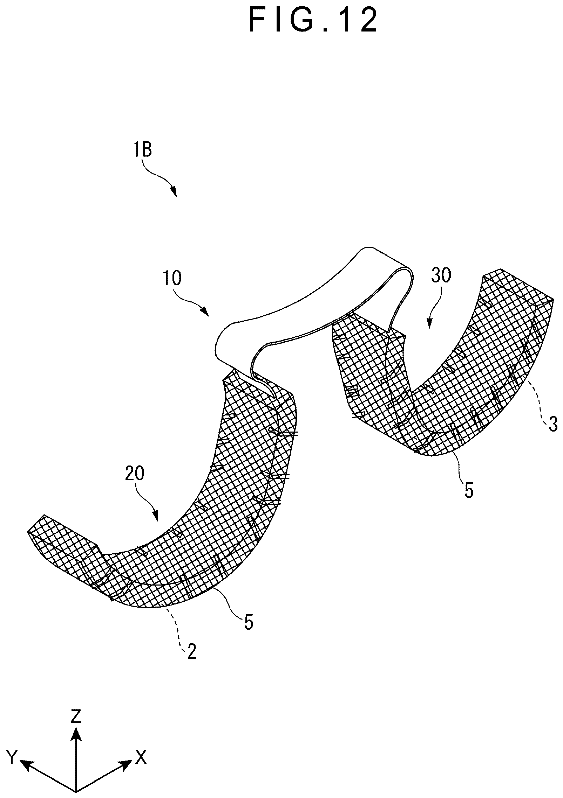

For instance, Fig. 12 illustrates a modification of the above-described first exemplary embodiment, in which a hallux valgus correction brace 1B includes two protective members 5. The protective members 5 are provided to cover the inner surfaces of the grooves 20 and 30 of the first insertion piece 2 and the second insertion piece 3.

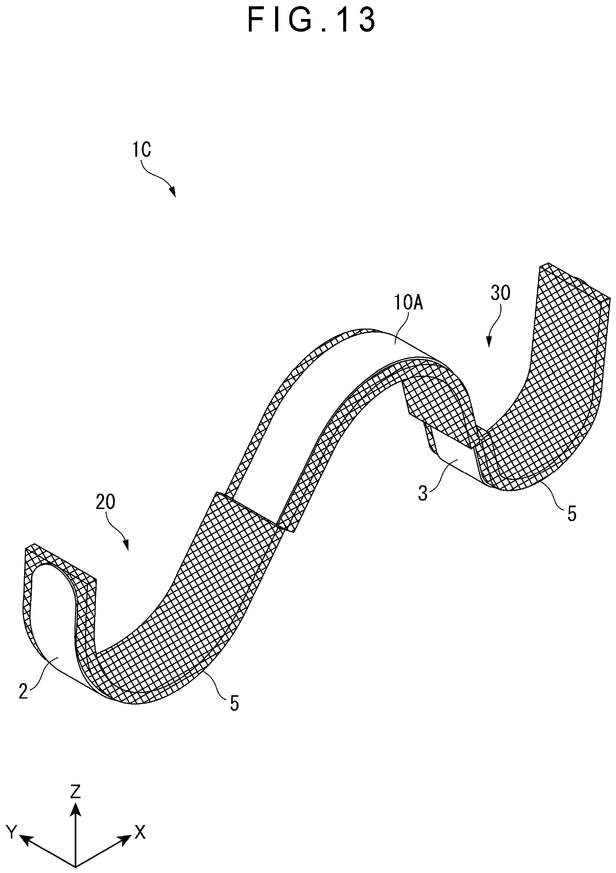

Fig. 13 illustrates a modification of the above-described second exemplary embodiment, in which a hallux valgus correction brace 1C includes three protective members 5. The protective members 5 are provided to cover the inner surfaces of the grooves 20, 30, and 40 of the first insertion piece 2A, the second insertion piece 3A, and the gap maintainer 4A.

In the modifications as described above, portions, which are likely to come into contact with the toes of the foot F of the user, of the elongated members 10 and 10A are covered with the protective members 5. The protective members 5 may be formed of any material with flexibility, such as nonwoven fabric. Moreover, the protective members 5 may be fixed to the elongated members 10 and 10A by any means such as an adhesive or sewing. The protective members 5, which prevent a direct contact of the foot F of the user with the metal elongated members 10 and 10A, can protect the foot F from friction, allergy, or the like.

It should be noted that portions, which come into contact with none of the toes of the foot F, of the elongated members 10 and 10A are exposed from the protective members 5 in Fig. 12 and Fig. 13, but the invention is not limited thereto. The whole of the elongated members 10 and 10A may be covered with the protective member(s).

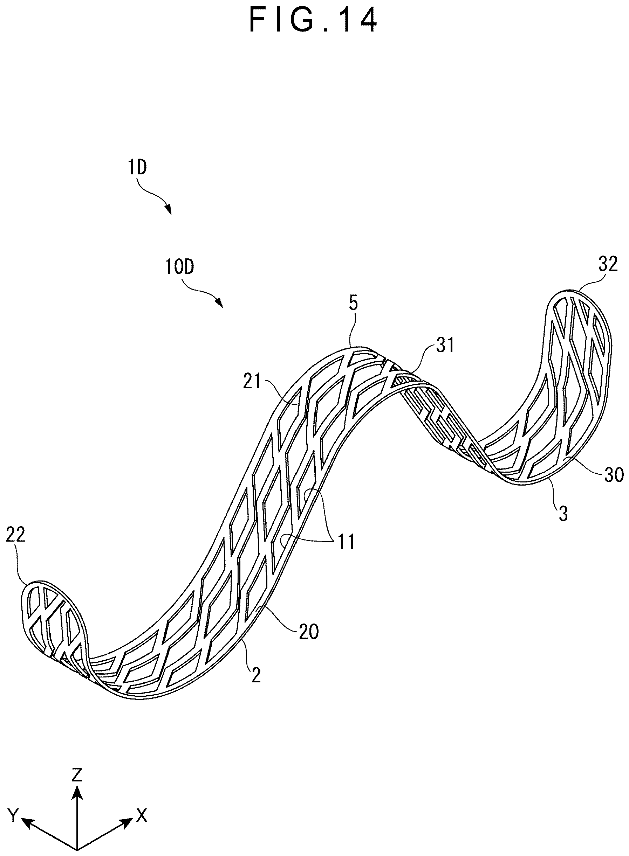

(2) In the above-described exemplary embodiments, the elongated members 10 and 10A may each have a mesh shape. For instance, Fig. 14 illustrates another modification of the above-described second exemplary embodiment, in which an elongated member 10D of a hallux valgus correction brace 1D has a mesh shape with a plurality of through holes 11. Such a configuration provides breathability of the hallux valgus correction brace 1D, and the foot F of the user can be inhibited from being sweaty even in a case where the hallux valgus correction brace 1D has a large Y-directional dimension.

(3) In the above-described exemplary embodiment, the elongated members 10 and 10A are each in a plate shape with a predetermined width and a predetermined thickness as being unfolded in the X-direction, but the invention is not limited thereto. For instance, the elongated members 10 and 10A may each have a plate shape varying in width or thickness depending on the portions. Alternatively, the elongated members 10 and 10A may each be in a linear shape other than the plate shape as being unfolded in the X-direction.

(4) In the above-described exemplary embodiments, the gap maintainers 4 and 4A are disposed on the +Z-side with respect to the outer ends 22 and 32 of the first insertion pieces 2 and 2A and the second insertion pieces 3 and 3A, but the invention is not limited thereto. For instance, at least one of the outer end 22 of the first insertion piece 2 (or 2A) or the outer end 32 of the second insertion piece 3 (or 3A) may extend on the +Z-side with respect to the gap maintainers 4 and 4A.

(5) In the above-described exemplary embodiments, the first insertion pieces 2 and 2A, the second insertion pieces 3 and 3A, and the gap maintainers 4 and 4A may each have any other shape, insofar as being integrally formed by the metal elongated members 10 and 10A, respectively.

For instance, each of the first insertion pieces 2 and 2A and the second insertion pieces 3 and 3A needs not be in a simple groove shape and may be formed in a shape surrounding a periphery of the corresponding toe of the foot F. Moreover, the gap maintainers 4 and 4A may provide a desired elastic force by being folded at a position(s) other than that described in the above-described exemplary embodiments. The elastic force may be an elastic force in a direction for at least the first insertion piece 2 (or 2A) and the second insertion piece 3 (or 3A) to move away from each other, and needs not contain the Z-directional component.

## Claims

1. A hallux valgus correction brace comprising:
a first insertion piece (2, 2A) configured to receive a big toe of a foot;
a second insertion piece (3, 3A) configured to receive a second toe or a middle toe of the foot; and
a gap maintainer (4, 4A) coupling the first insertion piece (2, 2A) and the second insertion piece (3, 3A), the gap maintainer (4, 4A) being configured to apply an elastic force to the first insertion piece (2, 2A) and the second insertion piece (3, 3A) in a direction away from each other, **characterized in that**
the first insertion piece (2, 2A), the second insertion piece (3, 3A), and the gap maintainer (4, 4A) are integrally formed by an elongated member (10, 10A, 10D) made of metal.

2. The hallux valgus correction brace according to claim 1, wherein a material of the elongated member (10, 10A, 10D) includes a nickel-titanium alloy.

3. The hallux valgus correction brace according to claim 2, wherein a shape recovery temperature of the nickel-titanium alloy is 32 degrees C or less.

4. The hallux valgus correction brace according to claim 1, wherein
assuming that a direction in which the first insertion piece (2, 2A) and the second insertion piece (3, 3A) are arranged side by side is defined as a foot-width direction and an insertion direction of the second toe or the middle toe into the second insertion piece (3, 3A) is defined as a foot-length direction,
the first insertion piece (2, 2A) and the second insertion piece (3, 3A) each form a groove (20, 30) opening on one side in an orthogonal direction orthogonal to each of the foot-width direction and the foot-length direction.

5. The hallux valgus correction brace according to claim 4, wherein
the first insertion piece (2, 2A) and the second insertion piece (3, 3A) each include an inner end (21, 31) and an outer end (22, 32) forming a pair of edges of the groove (20, 30), and
the gap maintainer (4, 4A) couples the inner end (21) of the first insertion piece (2, 2A) and the inner end (31) of the second insertion piece (3, 3A).

6. The hallux valgus correction brace according to claim 5, wherein the gap maintainer (4, 4A) is disposed on the one side in the orthogonal direction with respect to the outer end (22,32) of at least one of the first insertion piece (2, 2A) or the second insertion piece (3, 3A).

7. The hallux valgus correction brace according to claim 5, wherein the gap maintainer (4) includes:
a first projection (41) connected to the inner end (21) of the first insertion piece (2) and projecting toward the groove (20) of the first insertion piece (2);
a second projection (42) connected to the inner end (31) of the second insertion piece (3) and projecting toward the groove (30) of the second insertion piece (3); and
a coupler (43) coupling the first projection (41) and the second projection (42).

8. The hallux valgus correction brace according to claim 7, wherein the first projection (41) and the second projection (42) each have an arc shape.

9. The hallux valgus correction brace according to claim 7, wherein the coupler (43) has an arc shape curved to project on an other side in the orthogonal direction.

10. The hallux valgus correction brace according to claim 4, wherein the gap (4A) maintainer has an arc shape curved to project on the one side in the orthogonal direction.

11. The hallux valgus correction brace according to claim 4, further comprising:
one or more protective members (5) covering at least an inner surface of the groove (20, 30) of each of the first insertion piece (2, 2A) and the second insertion piece (3, 3A).

12. The hallux valgus correction brace according to claim 4, wherein the elongated member (10, 10A, 10D) has a plate shape with a predetermined width and a predetermined thickness as being unfolded in the foot-width direction.

13. The hallux valgus correction brace according to claim 12, wherein the elongated member (10D) has a mesh shape with a plurality of through holes (11).

## Patentansprüche

1. Eine Hallux-valgus-Korrekturorthese, umfassend:
ein erstes Einsteckteil (2, 2A), das zur Aufnahme des großen Zehs eines Fußes ausgebildet ist;
ein zweites Einsteckteil (3, 3A), das zur Aufnahme eines zweiten Zehs oder eines Mittelfußzehs des Fußes ausgebildet ist; und
einen Spalthalter (4, 4A), der das erste Einsteckteil (2, 2A) und das zweite Einsteckteil (3, 3A) miteinander verbindet, wobei der Spalthalter (4, 4A) so ausgebildet ist, dass er auf das erste Einsteckteil (2, 2A) und das zweite Einsteckteil (3, 3A) eine elastische Kraft in einer Richtung ausübt, die voneinander wegführt,
**dadurch gekennzeichnet, dass** das erste Einführteil (2, 2A), das zweite Einführteil (3, 3A) und der Spalthalter (4, 4A) einstückig aus einem länglichen Element (10, 10A, 10D) aus Metall geformt sind.

2. Die Hallux-valgus-Korrekturorthese nach Anspruch 1, wobei ein Material des länglichen Elements (10, 10A, 10D) eine Nickel-Titan-Legierung umfasst.

3. Die Hallux-valgus-Korrekturorthese nach Anspruch 2, wobei die Formrückstelltemperatur der Nickel-Titan-Legierung 32 °C oder weniger beträgt.

4. Die Hallux-valgus-Korrekturorthese nach Anspruch 1, wobei
unter der Annahme, dass eine Richtung, in der das erste Einsteckteil (2, 2A) und das zweite Einsteckteil (3, 3A) nebeneinander angeordnet sind, als Fußbreitenrichtung definiert ist und eine Einsteckrichtung des zweiten Zehs oder des mittleren Zehs in das zweite Einsteckteil (3, 3A) als Fußlängenrichtung definiert ist,
das erste Einsteckteil (2, 2A) und das zweite Einsteckteil (3, 3A) jeweils eine Nut (20, 30) bilden, die sich auf einer Seite in einer Richtung senkrecht zur Fußbreitenrichtung bzw. zur Fußlängenrichtung öffnet.

5. Die Hallux-valgus-Korrekturorthese nach Anspruch 4, wobei
das erste Einsteckteil (2, 2A) und das zweite Einsteckteil (3, 3A) jeweils ein inneres Ende (21, 31) und ein äußeres Ende (22, 32) aufweisen, die ein Kantenpaar der Nut (20, 30) bilden, und
der Spalthalter (4, 4A) das innere Ende (21) des ersten Einsteckstücks (2, 2A) und das innere Ende (31) des zweiten Einsteckstücks (3, 3A) miteinander verbindet.

6. Die Hallux-valgus-Korrekturorthese nach Anspruch 5, wobei der Spalthalter (4, 4A) auf der einen Seite in orthogonaler Richtung zum äußeren Ende (22, 32) mindestens eines der ersten Einsteckteile (2, 2A) oder der zweiten Einsteckteile (3, 3A) angeordnet ist.

7. Die Hallux-valgus-Korrekturorthese nach Anspruch 5, wobei der Spalthalter (4) umfasst:
einen ersten Vorsprung (41), der mit dem inneren Ende (21) des ersten Einsteckstücks (2) verbunden ist und in Richtung der Nut (20) des ersten Einsteckstücks (2) vorsteht;
einen zweiten Vorsprung (42), der mit dem inneren Ende (31) des zweiten Einsteckstücks (3) verbunden ist und in Richtung der Nut (30) des zweiten Einsteckstücks (3) vorsteht; und
ein Kupplungselement (43), das den ersten Vorsprung (41) und den zweiten Vorsprung (42) miteinander verbindet.

8. Die Hallux-valgus-Korrekturorthese nach Anspruch 7, wobei der erste Vorsprung (41) und der zweite Vorsprung (42) jeweils eine bogenförmige Gestalt aufweisen.

9. Die Hallux-valgus-Korrekturorthese nach Anspruch 7, wobei das Verbindungselement (43) eine Bogenform aufweist, die so gekrümmt ist, dass sie auf einer anderen Seite in orthogonaler Richtung vorsteht.

10. Hallux-valgus-Korrekturorthese nach Anspruch 4, wobei der Spalt (4A)-Halter eine Bogenform aufweist, die so gekrümmt ist, dass sie auf einer Seite in orthogonaler Richtung vorsteht.

11. Die Hallux-valgus-Korrekturorthese nach Anspruch 4, ferner umfassend:
ein oder mehrere Schutzelemente (5), die zumindest eine Innenfläche der Nut (20, 30) sowohl des ersten Einsteckstücks (2, 2A) als auch des zweiten Einsteckstücks (3, 3A) abdecken.

12. Die Hallux-valgus-Korrekturorthese nach Anspruch 4, wobei das längliche Element (10, 10A, 10D) eine Plattenform mit einer vorbestimmten Breite und einer vorbestimmten Dicke aufweist, wenn es in Fußbreitenrichtung entfaltet ist.

13. Hallux-valgus-Korrekturorthese nach Anspruch 12, wobei das längliche Element (10D) eine Netzform mit einer Vielzahl von Durchgangslöchern (11) aufweist.

## Revendications

1. Attelle de correction d'hallux valgus comprenant :
une première pièce d'insertion (2, 2A) configurée pour recevoir un gros orteil d'un pied ; une seconde pièce d'insertion (3, 3A) configurée pour recevoir un second orteil ou un orteil médian du pied ; et
un mainteneur d'écartement (4, 4A) reliant la première pièce d'insertion (2, 2A) et la seconde pièce d'insertion (3, 3A), le mainteneur d'écartement (4, 4A) étant configuré pour appliquer une force élastique à la première pièce d'insertion (2, 2A) et à la seconde pièce d'insertion (3, 3A) dans une direction les éloignant l'une de l'autre,
**caractérisée en ce que**
la première pièce d'insertion (2, 2A), la seconde pièce d'insertion (3, 3A) et le mainteneur d'écartement (4, 4A) sont formés d'un seul tenant par un élément allongé (10, 10A, 10D) constitué de métal.

2. Attelle de correction d'hallux valgus selon la revendication 1, dans laquelle un matériau de l'élément allongé (10, 10A, 10D) comporte un alliage nickel-titane.

3. Attelle de correction d'hallux valgus selon la revendication 2, dans laquelle une température de récupération de forme de l'alliage nickel-titane est de 32 °C ou moins.

4. Attelle de correction d'hallux valgus selon la revendication 1, dans laquelle en supposant qu'une direction dans laquelle la première pièce d'insertion (2, 2A) et la seconde pièce d'insertion (3, 3A) sont disposées côte à côte est définie comme une direction de largeur du pied et que la direction d'insertion du second orteil ou de l'orteil médian dans la seconde pièce d'insertion (3, 3A) est définie comme une direction de longueur du pied,
la première pièce d'insertion (2, 2A) et la seconde pièce d'insertion (3, 3A) forment chacune une rainure (20, 30) s'ouvrant sur un premier côté dans une direction orthogonale qui est orthogonale à chacune de la direction de largeur du pied et de la direction de longueur du pied.

5. Attelle de correction d'hallux valgus selon la revendication 4, dans laquelle la première pièce d'insertion (2, 2A) et la seconde pièce d'insertion (3, 3A) comportent chacune une extrémité interne (21, 31) et une extrémité externe (22, 32) formant une paire de bords de la rainure (20, 30), et
le mainteneur d'écartement (4, 4A) couple l'extrémité interne (21) de la première pièce d'insertion (2, 2A) et l'extrémité interne (31) de la seconde pièce d'insertion(3, 3A).

6. Attelle de correction d'hallux valgus selon la revendication 5, dans laquelle le mainteneur d'écartement (4, 4A) est disposé sur le premier côté dans la direction orthogonale par rapport à l'extrémité externe (22, 32) d'au moins l'une de la première pièce d'insertion (2, 2A) ou de la seconde pièce d'insertion (3, 3A).

7. Attelle de correction d'hallux valgus selon la revendication 5, dans laquelle le mainteneur d'écartement (4) comporte :
une première saillie (41) reliée à l'extrémité interne (21) de la première pièce d'insertion (2) et faisant saillie vers la rainure (20) de la première pièce d'insertion (2) ;
une seconde saillie (42) reliée à l'extrémité interne (31) de la seconde pièce d'insertion (3) et faisant saillie vers la rainure (30) de la seconde pièce d'insertion (3) ; et
un coupleur (43) couplant la première saillie (41) et la seconde saillie (42).

8. Attelle de correction d'hallux valgus selon la revendication 7, dans laquelle la première saillie (41) et la seconde saillie (42) ont chacune une forme en arc.

9. Attelle de correction d'hallux valgus selon la revendication 7, dans laquelle le coupleur (43) a une forme en arc courbée de manière à faire saillie sur un autre côté dans la direction orthogonale.

10. Attelle de correction d'hallux valgus selon la revendication 4, dans laquelle le mainteneur d'écartement (4A) a une forme en arc courbée de manière à faire saillie sur le premier côté dans la direction orthogonale.

11. Attelle de correction d'hallux valgus selon la revendication 4, comprenant en outre : un ou plusieurs éléments de protection (5) recouvrant au moins une surface interne de la rainure (20, 30) de chacune de la première pièce d'insertion (2, 2A) et de la seconde pièce d'insertion (3, 3A).

12. Attelle de correction d'hallux valgus selon la revendication 4, dans laquelle l'élément allongé (10, 10A, 10D) a une forme de plaque ayant une largeur prédéterminée et une épaisseur prédéterminée, comme étant dépliée dans la direction de largeur du pied.

13. Attelle de correction d'hallux valgus selon la revendication 12, dans laquelle l'élément allongé (10D) a une forme de treillis ayant une pluralité de trous traversants (11).
